Europäisches Patentamt

European Patent Office

Office européen des brevets

(19)

(11) Publication number: **0 136 620**
**B1**

# EUROPEAN PATENT SPECIFICATION

(12)

(45) Date of publication of patent specification: **13.01.88**

(21) Application number: **84111133.9**

(22) Date of filing: **18.09.84**

(51) Int. Cl.⁴: **C 07 K 13/00,** C 07 K 15/26,
C 12 P 21/02, C 12 N 15/00,
C 12 N 1/06, A 61 K 45/02

(54) **Homogeneous immune interferon fragment.**

(30) Priority: **20.09.83 US 534038**

(43) Date of publication of application:
**10.04.85 Bulletin 85/15**

(45) Publication of the grant of the patent:
**13.01.88 Bulletin 88/02**

(84) Designated Contracting States:
**AT BE CH DE FR GB IT LI LU NL SE**

(56) References cited:
**EP-A-0 077 670**
**EP-A-0 083 777**
**EP-A-0 088 540**

**NATURE, vol. 296, no. 5854, 18, March 1982,
New York (US); H.K.HOCHKEPPEL:
"Monoclonal antibody against human IFN-
gamma", pp. 258-259**

**O.A.NEUMÜLLER "Römpps Chemie-Lexikon",
7th ed., 1977, Franckh'sche Verlagshandlung,
Stuttgart (DE), pp. 3726-3727**

(73) Proprietor: **F. HOFFMANN-LA ROCHE & CO.
Aktiengesellschaft
CH-4002 Basel (CH)**

(73) Proprietor: **Takeda Chemical Industries, Ltd.
27, Doshomachi 2-chome Higashi-ku
Osaka-shi Osaka, 541 (JP)**

(72) Inventor: **Honda, Susumu
2-1, Tenjincho 2-chome
Takatsuki Osaka 569 (JP)**
Inventor: **Kung, Hsiang-Fu
21 West Lincoln Street
Verona, N.J. 07044 (US)**
Inventor: **Sugino, Hiromu 458-6,
Kamigoryonaka-machi
Muromachi-dori Kuramaguchi-sagaru
Higashi-iru Kamigyo-ku Kyoto 602 (JP)**

(74) Representative: **Lederer, Franz, Dr. et al
Van der Werth, Lederer & Riederer
Patentanwälte Lucile-Grahn-Strasse 22
D-8000 München 80 (DE)**

Courier Press, Leamington Spa, England.

**Description**

The present invention relates to a polypeptide having the following amino acid sequence

| | | | | | | | | | | | |
|---|---|---|---|---|---|---|---|---|---|---|---|
| Cys | Tyr | Cys | Gln | Asp | Pro | Tyr | Val | Lys | Glu | Ala | Glu |
| Asn | Leu | Lys | Lys | Tyr | Phe | Asn | Ala | Gly | His | Ser | Asp |
| Val | Ala | Asp | Asn | Gly | Thr | Leu | Phe | Leu | Gly | Ile | Leu |
| Lys | Asn | Trp | Lys | Glu | Glu | Ser | Asp | Arg | Lys | Ile | Met |
| Gln | Ser | Gln | Ile | Val | Ser | Phe | Tyr | Phe | Lys | Leu | Phe |
| Lys | Asn | Phe | Lys | Asp | Asp | Gln | Ser | Ile | Gln | Lys | Ser |
| Val | Glu | Thr | Ile | Lys | Glu | Asp | Met | Asn | Val | Lys | Phe |
| Phe | Asn | Ser | Asn | Lys | Lys | Lys | Arg | Asp | Asp | Phe | Glu |
| Lys | Leu | Thr | Asn | Tyr | Ser | Val | Thr | Asp | Leu | Asn | Val |
| Gln | Arg | Lys | Ala | Ile | His | Glu | Leu | Ile | Gln | Val | Met |
| Ala | Glu | Leu | Ser | Pro | Ala | Ala | Lys | Thr | Gly | Lys | |

which sequence may optionally contain an additional methionine residue at the amino terminus essentially free of other peptides and having substantially the same activity as gamma interferon and to pharmaceutical compositions containing such a polypeptide. The invention also relates to a process for producing such a polypeptide from a transformed microorganism capable of expressing IFN-γ. This process comprises extraction of the transformed microorganism after rupture of the cells, e.g. by sonification or chemical lysis, in the absence of a protease inhibitor and purification of the extract obtained using monoclonal antibodies to immune interferon.

Brief description of the figures

Figure 1 illustrates sodium dodecyl sulfate-polyacrylamide gel electrophoresis of purified recombinant human immune interferon under reducing condition (A, 0.7M β-mercaptoethanol) and non-reducing condition (B).

Figure 2 illustrates the comparison between the predicted amino acid sequence of recombinant human immune interferon and the actual DNA sequence of 15K and 18K rIFN-γ species.

Figure 3 illustrates the HPLC separation of C-terminal peptides of the 15K and 18K rIFN-γ after CNBr treatment.

The prior art has devised various methods for effecting the extraction and purification of the family of anti-viral proteins known as interferon. To date there are three known major classes of interferon: IFN-α (leukocyte), IFN-β (fibroblast) and IFN-γ (immune). Although the various interferon classes may be related in terms of anti-viral, anti-proliferative or structural arrangement, the prior art had so far been unable to devise a uniform method for the extraction and purification of all of these classes. Indeed, many of the processes useful for the extraction and purification of leukocyte interferon from a crude mixture of other proteins and cell debris would not work to extract and purify fibroblast or immune interferon from the same kind of preparation.

The extraction step in the purification processes of the prior art typically involved either the mechanical (i.e. sonification) or chemical lysis of microorganisms which have produced, often through recombinant techniques, the desired "foreign" protein. During this mechanical or chemical lysis procedure various cellular proteases are also released into the mixture. These proteases are then free to enzymatically act upon and degrade the "foreign" protein in the mixture.

It has further been reported that certain amino acid sequences on immune interferon are not essential for anti-viral activity. For instance, Capon et al. (reference 11) have reported that amino acids 136—146 of rIFN-γ are not essential for antiviral activity.

Description of the preferred embodiments

It has now been discovered in the case of immune interferon, that extraction and purification with any of the conventional techniques described in the prior art will not yield an entirely homogeneous preparation of immune interferon with an intact or complete amino acid sequence. Only recently has recombinant technology advanced to the point at which it is possible to obtain sufficient quantities of rIFN-γ so as to characterize it and determine its amino acid sequence. When conventional prior art techniques were utilized to extract rIFN-γ preparations and the amino acid sequence of purified material was determined, it was discovered that the purified preparation, in fact, comprised a variety of related protein species of different molecular weight. It has further now been discovered by amino acid sequencing that these proteins species are actually the intact sequence form of immune interferon in combination with a majority of proteolytic fragments of the intact sequence protein. Surprisingly, even though it has now been discovered that the prior art mixture contained both intact immune interferon protein and fragments thereof, it has been discovered that apparently a component of this mixture has retained biological activity.

The extraction of human rIFN-γ from transformed microorganisms producing and containing this protein can be carried out simply and efficiently with the use of sonification or mechanical lysis. However, it is believed that the rIFN-γ is degraded by proteases during the extraction step. Sonification of frozen cells yields a mixture of proteolytic products such as 15K rIFN-γ (MW about 15000 daltons; the compound of the present invention) which results from the removal of C-terminal amino acid residues Nos. 132—146. The

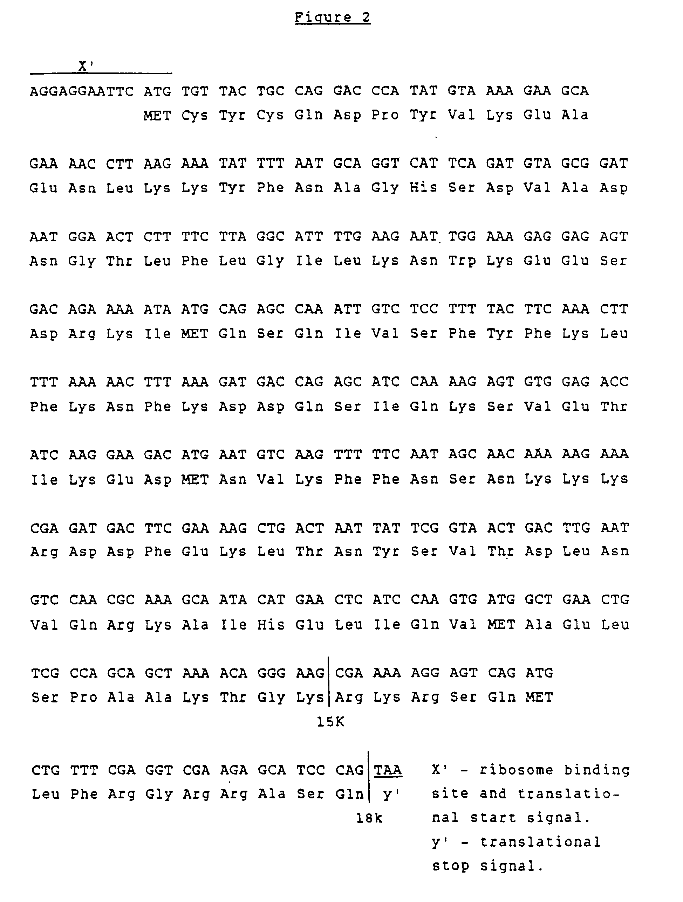

15K rIFN-γ after purification was found to be free of other proteolytic fragments and, significantly, had biological activity equal to that of the complete 18K rIFN-γ protein ($10^7$ units/mg) and comparable to that of homogeneous natural human immune interferon (reference 7). Amino acid compositions and N-terminal sequences of the 15K protein were consistent with that expected from the DNA sequence. The 15K rIFN-γ amino acid and corresponding DNA sequence is shown in Figure 2 along with the amino acid and corresponding DNA sequence of the complete immune interferon protein. The DNA base sequence of the complete, 18K human rIFN-γ as used throughout this specification is as described in reference 1.

Depending upon extraction procedures, different molecular weight (MW) species (about 15,000—18,000 daltons) of rIFN-γ have been separately purified. The purified interferon with apparent MW of 18,000 daltons on sodium dodecyl sulfate-polyacrylamide gel electrophoresis has been obtained by guanidine extraction. However, the lower 15,000 MW species immune interferon is preferably extracted by sonification in the absence of guanidine. The amino terminal sequence of both the 18K and 15K proteins were consistent with the sequence predicted from the DNA coding for the human immune interferon protein. It is also within the contemplation of this invention that the amino terminus of the immune interferon protein could begin with a methionine (Met) residue as additional amino terminal amino acid as opposed to cysteine (Cys) as shown in Figure 2.

The C-terminal sequences of the 18K and 15K protein were determined by analyzing and sequencing purified C-terminal peptide after CNBr treatment. The amino acid composition and sequence of the C-terminal peptide released from the 18K rIFN-γ matched with the predicted sequence of rIFN-γ indicating that the 18K species is the intact molecule. On the other hand, a different CNBr cleaved peptide was released from the C-terminus of 15K rIFN-γ. Based on amino acid and sequence analysis, this different peptide corresponded to amino acid residues No. 121—131 indicating that the 15K species was a proteolytic product.

In a preferred embodiment of this invention, the microorganism employed as the recipient in the fermentation procedures and unless otherwise noted, is the microorganism Escherichia coli K-12 strain 294 as described in British Patent Publication No. 2055382A which has been deposited with the American Type Culture Collection under ATCC Accession No. 31446, on October 28, 1978. Furthermore, all recombinant DNA work herein was performed in compliance with applicable guidelines of the National Institutes of Health.

The invention however, includes the use not only of E. coli K-12 strain 294, mentioned above, but also of other known equivalent E. coli strains such as E. coli MA210 or E. coli RR1 (ATCC No. 31343), or other microorganisms many of which are publicly available or are deposited and available from recognized microorganism depository institutions, such as the American Type Culture Collection (see e.g. the ATCC catalogue).

As used herein the term "interferon activity" refers to the characteristic antiviral and antigrowth activity characteristic of the interferons. The characteristic antiviral activity of rIFN can be determined using the cytopathic effect inhibition test described by Familletti, P. C., et al., Methods in Enzymology 78, 387 (1981). The characteristic antigrowth activity of rIFN can be determined using the procedure described by Evinger and Pestka in Methods in Enzymology, 79, 45 (1981).

Monoclonal antibodies were made against a synthetic polypeptide containing the last 16 amino acid residues of the C-terminal peptide of rIFN-γ. One of the monoclonal antibodies (Mo γ 2—11.1) was used for the purification of rIFN-γ. More specifically, the monoclonal antibodies and antibody affinity column of this invention were prepared as described in published European patent application No. 103 898.

In accordance with this invention the aforementioned novel immune interferon can be used for the same purposes as the other known interferons, e.g. in the prophylaxis or treatment of viral or neoplastic disorders or in the treatment of immunosupressive conditions. It may be administered in pharmaceutically acceptable oral, injectable or topical compositions and modes. Dosages and dose rates may parallel those currently being used in clinical applications of the known interferons, typically about $1—200×10^6$ units daily. These pharmaceutical compositions of the invention contain said immune interferon in association with a compatible pharmaceutically acceptable carrier material. Any conventional carrier material can be utilized. The carrier material can be an organic or inorganic inert carrier material suitable for enteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, preservatives, stabilizers, emulsifying agents, buffers and the like may be added in accordance with accepted practices of pharmaceutically compounding.

The pharmaceutical preparations can be made up in any conventional form including: a) a solid form for oral administration such as tablets, capsules, pills, powders, granules, and the like; b) a liquid form for oral administration such as solutions, syrups, suspensions, elixirs and the like; c) preparations for parenteral administration such as sterile solutions, suspensions or emulsions; and d) preparations for topical administrations such as solutions, suspensions, ointments, creams, gels, micronized powders, aerosols and the like. The pharmaceutical preparations may be sterilized and/or may contain adjuvants such as preservatives, stabilizers, wetting agents, emulsifiers, salts for varying the osmotic pressure and/or buffers.

Parenteral dosage forms may be infusions or injectable solutions which can be injected intravenously or intramuscularly. These preparations can also contain other medicinally active substances. Additional additives such as preservatives, stabilizers, emulsifying agents, buffers and the like may be added in accordance with accepted practices of pharmaceutical compounding.

Example 1
Synthesis of carrier protein-polypeptide complex used as antigen

The polypeptide H-Lys-Arg-Lys-Arg-Ser-Gln-Met-Leu-Phe-Arg-Gly-Arg-Arg-Ala-Ser-Gln-OH was coupled with thyroglobulin (hereinafter TG) according to the method of Goodfriend et al., Science *144*, 1334 (1964).

The peptide itself can be produced by conventional methods of peptide synthesis. Either of the solid phase and liquid phase method may be used, although the liquid phase synthetic method is advantageous in many cases. Such methods of peptide synthesis are described, for example, by Schröder and Lübke in "The Peptides", Vol. 1, Academic Press, New York, USA., 1966, or by Izumiya et al. in "Peptide Syntheses", Maruzen, Tokyo, Japan, 1975, or by Haruaki Yajima in "Experiments in Biochemistry", Vol. 1, pages 207—400, Tokyo Kagaku Dojin, 1977, and include, among others, the azide method, chloride method, acid anhydride method, mixed acid anhydride method, DCC method, active ester method, method using Woodward reagent K, carbodiimidazole method, oxidation-reduction method and DCC/additive (e.g. HONB, HOBt, HOSu), method.

For a detailed description of the preparation of the 131—146 fragment of IFN-γ see published European patent application No. 103 898.

2.5 mg of said polypeptide were mixed with 3.75 mg of TG and, following addition of 2 ml of 50 mM phosphate buffer, the mixture was stirred well in ice water. Thereto was gradually added drop by drop a solution of 30.4 mg of carbodiimide hydrochloride in 200 ml of distilled water. Thereafter, the mixture was stirred in ice water for 3 hours. After completion of the reaction, dialysis was performed against distilled water to a sufficient extent, followed by lyophilization to give 4.7 mg of a protein complex.

Example 2
Preparation of enzyme-linked antigen for enzyme immunoassay (EIA) for antibody detection

The enzyme-linked antigen for EIA was prepared according to Kitagawa et al., Journal of Biochemistry *79*, 233 (1976).

(i) Introduction of a maleimido group into the polypeptide

The polypeptide (350 nmoles) as obtained in Example 1 was dissolved in 1 ml of 100 mM phosphate buffer (pH 6.8), and the solution was added to a solution of 585 µg (1.75 µmoles) of N-(4-carboxycyclohexyl-methyl)maleimide N-hydroxy-succinimide ester in 70 µl of N,N-dimethylformamide. The mixture was stirred at 30° for 30 minutes. After completion of the reaction, fractionation was performed using a Sephadex G-25 column to give 185 nmoles of a polypeptide fraction with the maleimido group introduced therein.

(ii) Coupling of the maleimido-group-containing polypeptide with β-D-galactosidase

The maleimido-containing polypeptide (16.5 nmoles) was mixed with 3.3 nmoles of β-D-galactosidase. After 18 hours of reaction at 4°C, 412,5 nmoles of β-mercaptoethanol were added to terminate the reaction. The β-D-galactosidase—coupled polypeptide was fractionated on a Sepharose 6B column and used for the subsequent experiments.

Example 3
Immunization

Each of 6 female BALB/C mice aged 7 to 8 weeks was subcutaneously inoculated with 40 µg (on the basis of the protein) of the protein complex obtained in Example 1 as antigen in intimate admixture with Freund's complete adjuvant (primary immunization). Two weeks after the primary immunization, the mice were again subcutaneously inoculated with the antigen at the same dose as above in intimate admixture with Freund's incomplete adjuvant (secondary immunization). Further two weeks later, a third immunization was made in the same manner as in the secondary immunization. Six days after the third immunization, partial blood sampling was made from the mice and the serum antibody titers were determined by the EIA method described below (compare Immunopharmacology *1*, 3 [1978]). The mouse numbered γ-2 gave the highest antibody titer and was subjected to a final immunization by intravenous inoculation with 120 µg of the antigen dissolved in 0.5 ml of aqueous sodium chloride. The antibody titer data for each mouse are shown in Table 1.

4

TABLE 1
Antipeptide antibody titers in immunized mice

| Mouse No. | B/T (%) | | |
|---|---|---|---|
| | Primary immunization 1) | Secondary immunization 2) | Third immunization 3) |
| γ-1 | — 4) | N.D. | 24.5 |
| 2 | N.D. 5) | 19.3 | 35.3 |
| 3 | — | N.D. | 24.7 |
| 4 | N.D. | 1.3 | 1.7 |
| 5 | N.D. | 1.8 | 5.0 |
| 6 | — | N.D. | 0.8 |
| Normal mouse | 0.6 | 0.1 | N.D. |

1) Serum dilution ratio: 1/1000
2) Serum dilution ratio: 1/6300
3) Serum dilution ratio: 1/7800
4) —: Not detectable
5) N.D.: Not determined
B/T: (Bound enzyme activity/total added enzyme activity)×100

EIA method

The detection of antibody activity in the serum of mice immunized with the protein complex obtained in Example 2 or in the hybridoma supernatant was conducted by the EIA method [Immunology 1, 3 (1978)]. Thus, the serum or hybridoma supernatant was diluted with buffer A (20 mM $Na_2HPO_4$, 100 mM NaCl, 0.1% $NaN_3$, 1 mM $MgCl_2$, pH 7.0), a 100-µl portion of the dilution was mixed with 100 µl of the enzyme-linked polypeptide derivative (Example 2) and the reaction was allowed to proceed at 24°C for 24 hours. Thereafter, 100 µl of 3% cellulose coupled with rabbit anti-mouse IgG was added, and the reaction was allowed to proceed at 24°C for 4 hours. After completion of the reaction, the cellulose was washed well with buffer A containing 0.5% of Tween 20, then 500 µl of 20 µg/ml 4-methylumbelliferyl-β-D-galactoside was added and, after 2 hours of reaction of 37°C, 3 ml of 100 mM carbonate buffer (pH 10.5) were added to terminate the reaction. The fluorescence intensity was measured with a fluorometer (excitation: 365 nm; emission: 450 nm).

Example 4
Cell fusion

Three days after the final immunization as described in Example 3, the spleen was excised from the γ-2 mouse, filtered under pressure through a stainless mesh, and suspended in Eagle's minimum essential medium (MEM) to give a spleen cell suspension. For cell fusion, BALB/C mouse-derived P3-×63.Ag8.U1 (P3U1) myeloma cells were used [Current Topics in Microbiology and Immunology, 81, 1 (1978)]. Cell fusion was performed by the original method of Köhler and Milstein, Nature 256, 495—497 (1975). Thus, spleen cells and P3U1 cells were separately washed three times with serum-free MEM and mixed at a ratio of 5:1 (in number of cells). The mixture was centrifuged at 800 rpm for 15 minutes, whereby the cells were settled. After thorough removal of the supernatant, the sediment was lightly loosened, 0.3 ml of 45% polyethylene glycol (PEG) 6000 (Koch-Light) was added, and the mixture was allowed to stand in a warm water tank maintained at 37°C for 7 minutes so as to effect cell fusion. Thereafter, MEM was added thereto at a rate of 2 ml per minute. After addition of 12 ml in total of MEM, the resulting mixture was centrifuged at 600 rpm for 15 minutes, followed by removal of the supernatant. The cell sediment was suspended in RPMI-1640 medium supplemented with 10% fetal calf serum (RPMI1640-10FCS) in a concentration of $2×10^5$ P3U1 cells/ml and each of 144 wells on 24-well multidishes (Linbro) was seeded with 1 ml of the suspension. After seeding, the cells were incubated at 37°C in a 5% carbon dioxide gas incubator. After 24 hours, HAT-selective culture was started by adding RPMI1640-10FCS medium supplemented with HAT ($1×10^{-4}$ M hypoxanthine, $4×10^{-7}$ M aminopterin, $1.6×10^{-5}$ M thymidine) medium in an amount of 1 ml per well. The HAT-selective culture was continued while 1 ml of the old medium was replaced by 1 ml of HAT medium 3, 5 and 7 days after start of the culture. The growth of hybridomas was noted 10 to 14 days after cell fusion. When the culture broth turned yellow (about $1×10^6$ cells/ml), the supernatant was collected and examined for the presence of antibody by the EIA method. In this manner, supernatants from 141 wells in which hybridoma growth had been noted were examined. Two wells (γ 2-11 and γ 2-100) showed intense antibody activity and other two wells (γ 2-62 and γ 2-70) presented weak antibody activity.

Example 5
Cloning

Hybridomas from 3 wells (γ 2—11, γ 2—62 and γ 2—100) which were positive in antibody activity were cloned by the limiting dilution method. Thus, hybridoma cells were suspended in RPMI1640-20FCS in a concentration of at least 2 hybridoma cells/ml and the suspension was distributed in 0.1-ml portions into the wells on a 96-well microplate. In said distribution, $5 \times 10^5$ per well of BALB/C mouse thymocytes were added as feeder cells. As a result, cell proliferation was observed in about 2 weeks. The supernatant was then collected and examined for the presence of antibodies by the EIA method as described in Example 3. Antibody activity was noted in 8 of 19 clones from γ 2—11 well, in 3 of 54 clones from γ 2—62 well, and in 5 of 47 clones from γ 2—100 well (Table 2).

TABLE 2
Anti-peptide antibody activity of cloned hybridomas

| Hybridoma No. | B/T (%) |
|---|---|
| γ 2—11 | |
| 1 | 68 |
| 2 | 31 |
| 3 | 63 |
| 6 | 68 |
| 7 | 67 |
| 9 | 69 |
| 12 | 42 |
| 18 | 60 |
| γ 2—62 | |
| 14 | 20 |
| 16 | 21 |
| 34 | 16 |
| γ 2—100 | |
| 2 | 69 |
| 3 | 70 |
| 16 | 56 |
| 25 | 80 |
| 46 | 33 |
| Hyperimmune mouse serum | 35 |

Example 6
Binding capacity of monoclonal antibodies to IFN-γ

The binding capacity of monoclonal antibodies to IFN-γ was determined by the following method. To 300 μl of a 3% solution of cellulose coupled with rabbit antimouse IgG antibody, 300 μl of the culture supernatant of each of 2 or 3 cloned cell lines from each of γ 2—11, γ 2—62 and γ 2—100 wells was added, and the reaction was allowed to proceed at room temperature for 12 to 20 hours. Then, the cellulose was thoroughly washed with physiological saline, and 550 U/ml of IFN-γ obtained by the procedure mentioned below was added thereto. After 3 to 4 hours of reaction, the supernatant was collected and the IFN-γ obtained by the procedure mentioned below was added thereto. After 3 to 4 hours of reaction, the supernatant was collected and the IFN-γ activity therein was determined by the cytopathic effect (CPE) reading method using a microplate [Applied Microbiology *16*, 1706 (1968)]. Thus, 50 μl of MEM was placed in each well of a 96-well microplate and 50 μl of the IFN sample was added to the first well, followed by serial two-fold dilution. To each well thus prepared, 50 μl of a WISH cell suspension ($4 \times 10^5$ cells/ml) in 20% FCS-containing MEM was added, and incubation was conducted in a carbon dioxide gas incubator at 37°C for 24 hours. Thereafter, 50 μl of a vesicular stomatitis virus (New Jersey strain) preparation adjusted to a concentration of $2000 TCID_{50}$ ($TCID_{50}$: median tissue culture infecting dose) was added to each well and incubation was performed in a carbon dioxide incubator at 37°C. About 35 hours later, when cells in the IFN sample-free well showed 100% CPE, each well was microscopically observed for the estimation of CPE, and the reciprocal of the dilution factor for the IFN sample in that well in which 50% CPE was noted was referred to as the IFN titer.

The IFN-γ sample used was the supernatant collected 72 hours after stimulation of human peripheral lymphocytes with 40 µg/ml of concanavalin A and 15 ng/ml of 12-O—tetra-decanoylphorbol-13-acetate. Each ml of this culture supernatant contained 4400 units of human IFN-γ (heat and acid labile). If antibodies having binding capacity to IFN-γ are present in the cloned cell culture supernatant, then the added IFN-γ should bind to the antibodies on cellulose and reduction in IFN-γ activity of the supernatant should occur. As a result, for the clone γ 2—11, relatively intense binding activity to IFN-γ was noted and 50—75% of the added IFN-γ (550 U/ml) was bound to antibodies (Table 3).

TABLE 3
Absorption of IFN-γ activity by monoclonal antibodies

| Hybridoma culture | Residual IFN activity (U/ml) | |
|---|---|---|
| | Experiment 1 | Experiment 2 |
| γ 2—11.1 | 138 | 275 |
| γ 2—11.2 | 207 | N.D. |
| γ 2—11.6 | N.D. | 275 |
| γ 2—62.2 | 275 | 550 |
| γ 2—62.3 | 275 | 550 |
| γ 2—100.2 | 550 | N.D. |
| γ 2—100.3 | 550 | N.D. |
| — | 550 | 550 |

Example 7
Ascites formation by monoclonal antibody-producing hybridomas

Ascites formation was caused by intraperitoneal inoculation of BALB/C mice intraperitoneally pretreated with 0.5 ml of mineral oil with $1 \times 10^6$ γ 2—11.1 clone cells capable of producing antibodies having IFN-γ-binding activity. Ten days after intraperitoneal administration of hybridomas, the ascitic fluid was taken and examined for antibody activity up to $10^7$-fold dilution. While the antibody activity of the corresponding clone cell culture supernatant was detected up to $10^4$-fold dilution, the formation of ascites (ascitization) led to an about 1000 times increase in antibody activity.

Example 8
Monoclonal antibody purification

Using 4 ml of the ascitic fluid obtained in Example 7 as the starting material, monoclonal antibody purification was performed by the method of Staehelin et al. (Journal of Biological Chemistry 256, 9750, [1981]). Thus, the ascitic fluid was first centrifuged at 10,000 rpm for 15 minutes to remove fibrin-like substances therefrom and was then diluted with phosphate buffer-saline (PBS: 8.1 mM $NaH_2PO_4$, 1.5 mM $KH_2PO_4$, 2.7 mM KCl, 137 mM NaCl; pH 7.2) to a concentration at which the ultraviolet absorption at 280 nm ($A_{280}$) would range from 12 to 14. Thereafter, saturated aqueous ammonium sulfate was added to the diluted sample to a sulfate concentration of 47%. The mixture was stirred at 4°C for 60 minutes to effect salting out and was then centrifuged (10,000 rpm, 15 minutes) to give a precipitate. The precipitate was dissolved in 20 mM Tris buffer (pH 7.9) containing 50 mM NaCl and dialyzed against 2 liters of the same buffer. Two hours later, the dialyzing solution was replaced by a fresh 2-liter portion of the same solution and the dialysis was continued for further 15 hours. Thereafter, the precipitate was removed by centrifugation at 10,000 rpm for 15 minutes, and the supernatant was adjusted to a concentration such that the $A_{280}$ value became 20—30. This sample was subjected to fractionation on a DEAE-cellulose column (8 ml, Whatman $DE_{52}$) equilibrated with a sufficient amount of Tris buffer (pH 7.9) containing 50 mM NaCl, elution being made with Tris buffer containing 50 mM NaCl at a flow rate of 1.5 ml/minutes. Under these conditions, the antibody activity was detected mainly in effluent fractions. Confirmation that the purified sample is antibody was made by the SDS-PAGE method as described by Laemmli et al., Nature 227, 680 (1970). Thus, some of the fractions obtained by ammonium sulfate salting out and DEAE-cellulose fractionation were each subjected to reduction with 2-mercaptoethanol, followed by 17% SDS gel electrophoresis at 30 volts for 24 hours. In agreement with the antibody activity peaks, two bands were noted at positions corresponding to molecular weights of about 55 kilodaltons (H chain) and about 28

7

kilodaltons (L chain). The thus purified antibody fraction 17 was examined for IFN-γ-binding activity by adding IFN-γ (2200 U/ml). It was thus found that about 50% of IFN-γ was bound to the antibody (Table 4).

TABLE 4

| Sample | Dilution | Residual IFN activity (U/ml) |
|---|---|---|
| γ 2—11.1 fraction 17 | $10^{-1}$ | 1100 |
| | $10^{-2}$ | 1100 |
| | $10^{-3}$ | 2200 |
| | $10^{-4}$ | 2200 |
| Anti-IgE monoclonal antibody | $10^{-1}$ | 2200 |
| | $10^{-2}$ | 2200 |
| | $10^{-3}$ | 2200 |
| | $10^{-4}$ | 2200 |

Example 9

Subclass to which monoclonal antibodies belong

Purified antibody fraction 17 (Example 8) was diluted 10 times and subjected to immuno-precipitation reaction in agar (Ouchterlony test: Immunological Methods, Gel-Diffusion Techniques, Blackwell, Oxford, 1964) using goat antimouse IgG1, G2a, G2b and G3 antibodies (Miles) so that the IgG subclass to which γ 2—11.1 monoclonal antibodies might belong could be identified. A single distinct band was found between the monoclonal antibody and the goat anti-mouse IgG2b antibody, while no band formation was noted between the monoclonal antibody and other anti-antibodies. Accordingly, said monoclonal antibody was found to belong to IgG2b (Table 5).

TABLE 5
Monoclonal antibody subclass

| Antigen | Antibody | Precipitation curve |
|---|---|---|
| Monoclonal antibody of the present invention (fraction 17) | Anti-IgG1 | — |
| " | Anti-IgG2a | — |
| " | Anti-IgG2b | + |
| " | Anti-IgG3 | — |

Example 10

Twenty-five ml (65.3 mg) of the monoclonal antibody from the effluent fractions as purified by the procedure of Example 8 was dialyzed overnight against 0.1M NaHCO$_3$ (pH 8.3). Separately, 25 ml of AFFI-GEL 10 (Bio-Rad) was thoroughly washed with water using a glass filter, suspended in 0.1M NaHCO$_3$ (pH 8.3) and mixed with the above antibody. The mixture was stirred gently at 4°C for 4 hours to effect the reaction, and then allowed to stand at 4°C overnight. The AFFI-GEL 10 was washed well with 0.1M NaHCO$_3$ (pH 8.3) using a glass filter. To the gel were added 25 ml of a solution (pH 8.0) containing 0.1M ethanolamine and 0.15M NaCl. The mixture was shaken at 4°C for an hour so as to block possibly remaining unreacted active groups. Then, the gel was washed well with PBS, and suspended in 25 ml of 0.1% NaN$_3$-containing PBS. The suspension was stored at 4°C. Based on the amount of the added antibody and the amount of the antibody in the recovered filtrate, it was found that the antibody was conjugated to the gel in a proportion of 2.35 mg/ml of gel. A column was packed with the reaction product obtained in this manner and used as an antibody column.

Example 11

E. coli RR1 (pRK148cl$_{ts}$, pRC231/IFI-900) (construction of this recombinant organism is described in detail in European patent application publication No. 99084) was used for rIFN-γ production. Plasmids pRK248cl$_{ts}$ and pRC231/IFI-900 contained temperature sensitive Lambda repressor and IFN-γ genes respectively. Expression of rIFN-γ gene was under the control of the P$_L$ promoter.

Overnight cultures of E. coli RR1 (pRK248cl$_{ts}$, pRC231/IFI-900) were grown in LB broth at 30°C. One liter of the overnight culture was diluted to 10 liters with minimal M-9 medium containing casamino acids. At logarithmic growth, the culture was shifted from 30° to 42°C and continued to grow at 42°C for 2—3 hours. Bacteria were harvested by centrifugation and the bacterial pellets were stored at −20°C until used. All fermentations and procedures were performed in accordance with recombinant DNA guidelines of the National Institutes of Health.

Monoclonal antibodies were made against synthetic 131—146 C-terminal peptide of rIFN-γ in the manner described above. Monoclonal antibody Mo γ 2—11.1 was used for the purification of rIFN-γ. The monoclonal antibody column was prepared as described in Example 10.

Carboxymethylation of rIFN-γ by $^{14}$C-iodoacetic acid was carried out in the presence of 6M guanidine-HCl as described in reference (3). Excess reagent was removed by HPLC on C8 reverse-phase column. Carboxypeptidase digestion was performed in 0.2M NH$_4$HCO$_3$ as described in reference (4).

The rIFN-γ was treated with CNBr (100-fold molar excess over methionine) in 70 percent formic acid as described in reference (5). CNBr peptides were separated by HPLC on a C-18 reverse-phase column. A linear gradient of 0 to 70 percent of CH$_3$CN in 0.1% trifluoroacetic acid was used for peptide elution.

Protein or peptide samples were hydrolyzed for 20—24 hours in sealed, N$_2$-flushed, evacuated tubes in constant boiling HCl containing 4% thioglycolic acid. Amino acid analyses were performed using a fluorescamine amino acid analyzer as described in reference (6).

An ABI (Applied Biosystem, Inc.) gas-phase sequencer 470A was used for sequence analyses of carboxymethylated proteins as described in reference (7). Samples of PTH-amino acids were identified by reverse-phase HPLC on an ultrasphere ODS column as described in reference (8).

All purification steps were carried out at 4°C. Frozen cells (25 g) were suspended in three volumes (75 ml) of 7M guanidine-HCl (pH 7). The mixture was stirred for 1 h and then centrifuged for 1 h at 30,000×g. The supernatant was diluted 10-fold with Dulbecco's phosphate buffered saline (PBS) or 0.15M sodium borate buffer (pH 9.5) and then centrifuged for 30 min. at 30,000×g. Alternatively, frozen cells (25 g) were suspended in 1.5 volumes (37.5 ml) of 0.15M sodium borate buffer (pH 9.5) and stirred for 1 h. The mixture was sonicated 5 times for 30 seconds and then centrifuged for 1 h at 30,000×g. The supernatants from either guanidine-HCl extraction or sonification were mixed for 1 h on a rotating shaker with 25 ml silica and prewashed with PBS. The mixture was poured onto a column and the column was washed with 20—30 column volumes of 1M NaCl. The column was then eluted with 0.5M tetramethylammonium chloride in 0.01M sodium borate buffer (pH 8.0). Interferon activity was eluted in about 200 ml and separated into 4 pools. Each pool was loaded onto a monoclonal antibody (γ 2—11.1) affinity column (4 ml bed volume) equilibrated with PBS. After washing with 10 column volumes of PBS buffer, the column was eluted with either 1M guanidine-HCl or 50% ethylene glycol containing 1M NaCl and 20 mM sodium phosphate buffer (pH 7.0). Interferon activity was eluted in the first 20 ml.

SDS-PAGE was performed as described by Laemmli (reference 9). Protein was determined by fluorescamine analysis with crystalline bovine serum albumin as the reference standard. Interferon activity was determined by a cytopathic effect inhibition assay with vesicular stomatitis virus and human WISH cells as reported in reference 10. All interferon titers are expressed in reference units/ml calibrated against the reference standard of partially purified human immune interferon.

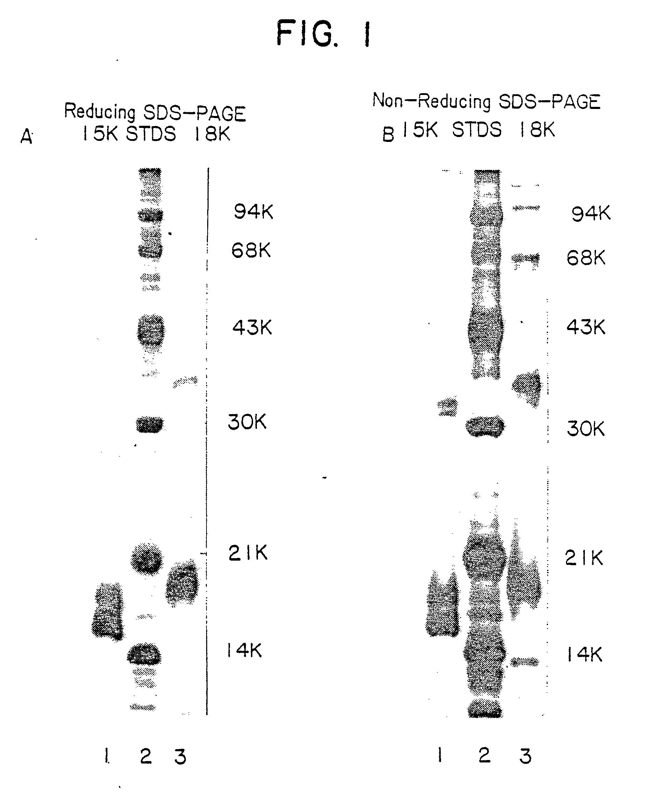

A summary of the extraction purification procedure is presented in Table 6 (page 24). The overall recovery was 25—32% and the purification was about 100 to 769 fold with an average specific activity of 1×10$^7$ units/mg. Total yield of rIFN-γ was 3—4 times higher with guanidine extraction. SDS-PAGE of the last stage of purification is shown in Figure 1. The material purified from guanidine extraction showed a single band at about 18,000 daltons (18K rIFN-γ) whereas the material from the sonification procedure yielded a major band at about 15,000 daltons (15K rIFN-γ) and a minor band at about 17,000 daltons (Figure 1A). On non-reducing gel, dimers and oligomers of rIFN-γ were formed (Figure 1B).

The 18K rIFN-γ was homogeneous and the amino acid composition was consistent with that predicted from the DNA sequence. Amino acid compositions of the 15K and 18K rIFN-γ are given in Table 7. Several hundred picomoles of reduced and carboxymethylated 15K and 18K protein underwent Edman degradation in an automatic gas-phase protein/peptide sequencer. The amino acid N-terminal sequences of the first 32 and 26 residues of the 15K and 18K proteins respectively were in accord with that predicted by the DNA sequence (Figure 2). $^{14}$C-Carboxymethylated cysteines were detected in the first and third cycles of sequence analyses. No N-terminal methionine was detected. N-terminal sequence analysis of the 18K and 15K rIFN-γ demonstrated that the sequence of this area of both proteins is identical to that predicted from the DNA sequence. The C-terminal peptides have also been characterized to determine whether any deletions or changes occurred in this region. Amino acid analysis of carboxypeptidase A (CPA) digestion mixture indicated that serine and/or glutamine (C-terminal amino acids) were released from the 18K rIFN-γ, whereas the 15K rIFN-γ was not digested by CPA under same conditions. Since Ser-Gln is the C-terminal

sequence of rIFN-γ the 18K species appeared to have the intact C-terminus while the 15K species has a different C-terminal residue (Lys) which is not cleaved by CPA.

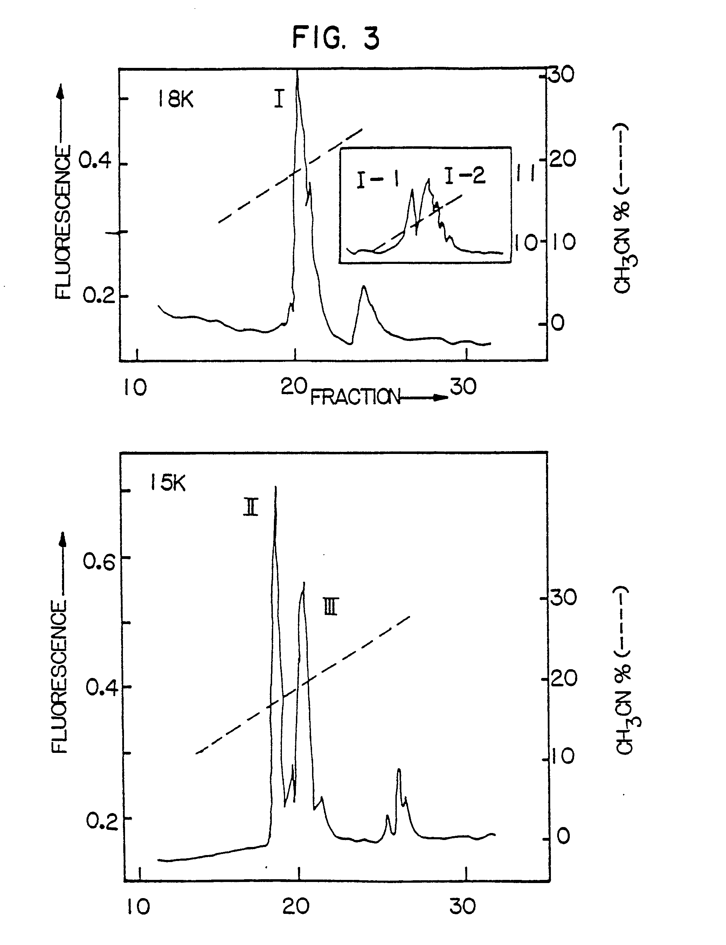

The C-terminal residues predicted from the DNA sequences were further confirmed by analyzing and sequencing the C-terminal peptides after CNBr treatment. C-terminal peptides were separated on the HPLC C-18 reverse-phase column (Figure 3). A sharp peptide peak (peak II), eluted from the early part of the gradient, was obtained from the 15K rIFN-γ and this peptide was absent from the CNBr digestion mixture of the 18K rIFN-γ. Amino acid analysis of this peptide indicated that this peptide has no homoserine or homoserine lactone and therefore must be the C-terminal CNBr peptide of the 15K protein. Based on amino acid analysis (Table 8), this peptide corresponded to amino acid residues No. 121—131 of IFN-γ (no arginine was detectable). The sequence of the 11 amino acids was confirmed by sequence analysis (Figure 2). In the case of 18K rIFN-γ, a relatively broad peak was obtained in the early part of the elution. This peak was further separated into two peaks by a shallow gradient. The amino acid analyses indicated that the first peak is the CNBr C-terminal peptide of the 18K protein (Table 8) and the amino acid composition corresponds to amino acid residues No. 138—146. The sequence of the 9 amino acids was verified by sequence determination (Figure 2). The C-terminal amino acid of the 15K protein was determined to be lysine.

These results indicate that the 18K species was the intact rIFN-γ molecule, whereas the 15K species was a proteolytic product. The peptide bond between amino acid residues No. 131 and No. 132 (Lys-Arg) was cleaved on the 15K species.

Example 12

The 15K fragment of Immune interferon was purified to homogeneity from the mixture containing other fragments, i.e. the 17K fragment, by the following additional procedures. A sonification supernatant (protein 10.3 mg; specific activity, $0.9 \times 10^6$ U/mg) which has been purified through the monoclonal antibody affinity column was further purified by SDS-PAGE gel electrophoresis (16 slab gels; acrylamide 12.5%; SDS, 0.1%; thickness, 3—5 mm). One slot of the gel was first stained with 0.1% Coomassie Brilliant Blue R-250 then destained with 10% acetic acid in 15% methanol. The corresponding 15K gel bands of other slots on the gel were cut out, crushed and stirred in 0.1% SDS at 4°C for 2 days. The solution was then filtered on filter paper. The filtrate was lyophilized and then combined with 6 volumes of ethanol. The resultant precipitate was dissolved in 0.1% SDS to yield a solution of homogeneous 15K monomer component (protein, 1.1 mg; specified activity, $0.3 \times 10^6$ U/mg).

TABLE 6
Purification of rIFN-γ

| Purification step | Total protein mg | Total activity units | Specific activity unit/mg | Purification -fold | Yield % |
|---|---|---|---|---|---|
| I. Guanidine extraction | | | | | |
| Supernatant | 2.806 | $2.5 \times 10^8$ | $9 \times 10^4$ | — | 100 |
| Silica | 98 | $1.0 \times 10^8$ | $1 \times 10^6$ | 11 | 40 |
| Monoclonal antibody | 8 | $0.8 \times 10^8$ | $1 \times 10^7$ | 110 | 32 |
| II. Sonification | | | | | |
| Supernatant | 6.136 | $8.0 \times 10^7$ | $1.3 \times 10^4$ | — | 100 |
| Silica | 87 | $4.5 \times 10^7$ | $5.2 \times 10^6$ | 400 | 56 |
| Monoclonal antibody | 2 | $2.0 \times 10^7$ | $1.0 \times 10^7$ | 769 | 25 |

TABLE 7
Amino acid compositions of 15K and 18K rIFN-γ (residue numbers)

| | 18K (1—146) | 15K (1—131) |
|---|---|---|
| Asp | 20.9 (20) | 19.9 (20) |
| Thr | 5.1 (5) | 4.9 (5) |
| Ser | 9.8 (11) | 6.7 (9) |
| Glu | 18.5 (18) | 15.1 (16) |
| Pro | * (2) | * (2) |
| Gly | 5.9 (5) | 5.6 (4) |
| Ala | 8.2 (8) | 7.3 (7) |
| Cys | * (2) | * (2) |
| Val | 9.1 (8) | 9.1 (8) |
| Met | 4.7 (4) | 3.8 (3) |
| Ile | 7.2 (7) | 6.6 (7) |
| Leu | 10.5 (10) | 9.6 (9) |
| Tyr | 5.5 (5) | 4.8 (5) |
| Phe | 10.3 (10) | 8.2 (9) |
| His | 1.8 (2) | 2.5 (2) |
| Lys | 19.9 (20) | 16.9 (19) |
| Arg | 8.6 (8) | 5.6 (3) |
| Trp | * (1) | * (1) |

Figures in parenthesis indicate the predicted amount of residues from DNA sequence.
*Values not determined.

TABLE 8

CNBr C-terminal peptides of 15K and 18K

|  | 15K | 18K |
|---|---|---|
| Thr | 0.9 (1) |  |
| Ser | 1.1 (1) | 0.84 (1) |
| Glu | 1.1 (1) | 1.1 (1) |
| Pro | * (1) |  |
| Gly | 1.5 (1) | 1.3 (1) |
| Ala | 2.4 (3) | 1.1 (1) |
| Leu | 1.0 (1) | 1.1 (1) |
| Phe |  | 1.0 (1) |
| Lys | 1.9 (2) | 2.8 (3) |
| Positions in sequence | 121—131 | 138—146 |

Figures in parenthesis indicate the predicted residue number from DNA sequence.
*not determined.

References

1. Gray, P. W., et al., Nature *295*, 503—508 (1982)
2. Stahelin, T., et al., J. Biol. Chem. *256*, 9750—9754 (1981)
3. Allen, G., Sequencing of Protein and Peptides, pp. 30—31 (1981), North-Holland Publishing Co., Amsterdam, New York
4. Amber, R. P., Methods in Enzymology *11*, 436—445 (1967)
5. Wolfe, R. A. and Stein, S., Modern Methods in Pharmacology, pp. 55—77 (1982), Alan R. Liss, Inc., New York, NY.
6. Stein, S. and Brink, L., Methods in Enzymology, *79*, 20—25 (1981)
7. Hewick, R. M., Hunkapillar, M. W., Hodd, L. E. and Dreyer, W. I., J. Biol. Chem. *256*, 7990—7997 (1981)
8. Hawke, D., Yuan, P-M., and Shively, J. E., Anal. Biochem. *120*, 302—311 (1982)
9. Laemmli, U.K., Nature *227*, 680—685 (1970)
10. Rubinstein S., Familletti, P. C., and Pestka, S., J. Virol, *37*, 755—758 (1981)
11. Capon. D. J., Leung, D. W., Hitzeman, R. A., Perry, L. J., Kohr, W. J., Gray, P. W., Derynck, R., and Goeddel, D. V., The Third Annual International Congress for Interferon Research, Miami, Fla. (1982).

**Claims for the Contracting States: BE, CH, DE, FR, GB, IT, LI, LU, NL, SE**

1. A polypeptide having the following amino acid sequence

```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

which sequence may optionally contain an additional methionine residue at the amino terminus essentially free of other peptides and having substantially the same activity as gamma interferon.

2. The polypeptide of claim 1 as a homogeneous peptide.

3. A polypeptide as claimed in claim 1 or claim 2 when used as a therapeutic agent.

4. A process for producing a polypeptide having the following amino acid sequence

```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro · Ala  Ala  Lys  Thr  Gly  Lys
```

which sequence may optionally contain an additional methionine residue at the amino terminus essentially free of other peptides and having substantially the same activity as gamma interferon which process comprises extracting transformed microorganisms capable of expressing immune interferon after rupture of the cells in the absence of a protease inhibitor and purifying the extract obtained using monoclonal antibodies to immune interferon.

5. A process as claimed in claim 4 wherein cell rupture is achieved by sonification.

6. Pharmaceutical compositions containing as active agent a polypeptide as claimed in claim 1 or claim 2 and an inert pharmaceutically acceptable carrier.

**Claims for the Contracting State: AT**

1. A process for producing a polypeptide having the following amino acid sequence

```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

which sequence may optionally contain an additional methionine residue at the amino terminus essentially free of other peptides and having substantially the same activity as gamma interferon which process comprises extracting transformed microorganisms capable of expressing immune interferon after rupture of the cells in the absence of a protease inhibitor and purifying the extract obtained using monoclonal antibodies to immune interferion.

2. A process as claimed in claim 1 wherein cell rupture is achieved by sonification.

**Patentansprüche für die Vertragsstaaten: BE, CH, DE, FR, GB, IT, LI, LU, NL, SE**

1. Ein Polypeptid mit der folgenden Aminosäuresequenz

```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

die gegebenenfalls einen zusätzlichen Methioninrest am Aminoende enthalten kann, im wesentlichen frei von anderen Peptiden und mit im wesentlichen gleicher Aktivität wie Gamma Interferon.

2. Das Polypeptide gemäss Anspruch 1 als ein homogenes Peptid.

3. Ein Polypeptid gemäss Anspruch 1 oder 2 als ein therapeutisches Agens verwendet.

4. Ein Verfahren zur Herstellung eines Polypeptids mit der folgenden Aminosäuresequenz

```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
```

```
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

die gegebenenfalls einen zusätzlichen Methioninrest am Aminoende enthalten kann, im wesentlichen frei von anderen Peptiden und mit im wesentlichen gleicher Aktivität wie Gamma Interferon, dadurch gekennzeichnet, dass man transformierte Mikroorganismen, die befähigt sind Immun-Interferon zu exprimieren, nach dem Aufbrechen der Zellen in Abwesenheit eines Protease Inhibitors extrahiert und den erhaltenen Extrakt mittels monoklonalen Antikörpern gegen Immun-Interferon reinigt.

5. Ein Verfahren gemäss Anspruch 4, bei dem das Aufbrechen der Zellen mittels Beschallung bewerkstelligt wird.

6. Pharmazeutische Zusammensetzungen enthaltend ein Polypeptid gemäss Anspruch 1 oder 2 als aktives Agens und ein inaktives, pharmazeutisch geeignetes Trägermaterial.

**Patentansprüche für den Vertragsstaat: AT**

1. Ein Verfahren zur Herstellung eines Polypeptids mit der folgenden Aminosäuresequenz
```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

die gegebenenfalls einen zusätzlichen Methioninrest am Aminoende enthalten kann, im wesentlichen frei von anderen Peptiden und mit im wesentlichen gleicher Aktivität wie Gamma Interferon, dadurch gekennzeichnet, dass man transformierte Mikroorganismen, die befähigt sind Immun-Interferon zu exprimieren, nach dem Aufbrechen der Zellen in Abwesenheit eines Protease Inhibitors extrahiert und den erhaltenen Extrakt mittels monoklonalen Antikörpern gegen Immun-Interferon reinigt.

2. Ein Verfahren gemäss Anspruch 1, bei dem das Aufbrechen der Zellen mittels Beschallung bewerkstelligt wird.

**Revendications pour les Etats Contractants: BE, CH, DE, FR, GB, IT, LI, LU, NL, SE**

1. Polypeptide ayant la séquence d'acides aminés suivante:
```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

laquelle séquence peut éventuellement contenir un résidu méthionine additionnel à l'extrémité amino essentiellement dépourvu d'autres peptides et ayant essentiellement la même activité que l'interféron gamma.

2. Polypeptide de la revendication 1 sous forme de peptide homogène.

3. Polypeptide selon la revendication 1 ou la revendication 2 lorsqu'il est utilisé comme agent thérapeutique.

4. Procédé de production d'un polypeptide ayant la séquence d'acides aminés suivante:
```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
```

```
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

laquelle séquence peut éventuellement contenir un résidu méthionine additionnel à l'extrémité amino essentiellement dépourvu d'autres peptides et ayant essentiellement la même activité que l'interféron gamma, lequel procédé comprend l'extraction de microorganismes transformés capables d'exprimer l'interféron immun apprès rupture des cellules en l'absence d'un inhibiteur de protéase et la purification de l'extrait obtenu en utilisant des anticorps monoclonaux anti-interféron immun.

5. Procédé selon la revendication 4 dans lequel la rupture des cellules s'effectue par traitement aux ultra-sons.

6. Compositions pharmaceutiques contenant comme agent actif un polypeptide selon la revendication 1 ou la revendication 2 et un support inerte pharmaceutiquement acceptable.

**Revendications pour l'Etat Contractant: AT**

1. Procédé de production d'un polypeptide ayant la séquence d'acides aminés suivante:

```
Cys  Tyr  Cys  Gln  Asp  Pro  Tyr  Val  Lys  Glu  Ala  Glu
Asn  Leu  Lys  Lys  Tyr  Phe  Asn  Ala  Gly  His  Ser  Asp
Val  Ala  Asp  Asn  Gly  Thr  Leu  Phe  Leu  Gly  Ile  Leu
Lys  Asn  Trp  Lys  Glu  Glu  Ser  Asp  Arg  Lys  Ile  Met
Gln  Ser  Gln  Ile  Val  Ser  Phe  Tyr  Phe  Lys  Leu  Phe
Lys  Asn  Phe  Lys  Asp  Asp  Gln  Ser  Ile  Gln  Lys  Ser
Val  Glu  Thr  Ile  Lys  Glu  Asp  Met  Asn  Val  Lys  Phe
Phe  Asn  Ser  Asn  Lys  Lys  Lys  Arg  Asp  Asp  Phe  Glu
Lys  Leu  Thr  Asn  Tyr  Ser  Val  Thr  Asp  Leu  Asn  Val
Gln  Arg  Lys  Ala  Ile  His  Glu  Leu  Ile  Gln  Val  Met
Ala  Glu  Leu  Ser  Pro  Ala  Ala  Lys  Thr  Gly  Lys
```

laquelle séquence peut éventuellement contenir un résidu méthionine additionnel à l'extrémité amino essentiellement dépourvu d'autres peptides et ayant essentiellement la même activité que l'interféron gamma, lequel procédé comprend l'extraction de microorganismes transformés capables d'exprimer l'interféron immun après rupture des cellules en l'absence d'un inhibiteur de protéase et la purification de l'extrait obtenu en utilisant des anticorps monoclonaux anti-interféron immun.

2. Procédé selon la revendication 1 dans lequel la rupture des cellules d'effectue par traitement aux ultra-sons.

# FIG. 1

Reducing SDS—PAGE
A  15K STDS 18K

Non—Reducing SDS—PAGE
B 15K STDS 18K

94K

68K

43K

30K

21K

14K

1  2  3

1  2  3

## Figure 2

<u>X'</u>

AGGAGGAATTC ATG TGT TAC TGC CAG GAC CCA TAT GTA AAA GAA GCA
            MET Cys Tyr Cys Gln Asp Pro Tyr Val Lys Glu Ala


GAA AAC CTT AAG AAA TAT TTT AAT GCA GGT CAT TCA GAT GTA GCG GAT
Glu Asn Leu Lys Lys Tyr Phe Asn Ala Gly His Ser Asp Val Ala Asp


AAT GGA ACT CTT TTC TTA GGC ATT TTG AAG AAT TGG AAA GAG GAG AGT
Asn Gly Thr Leu Phe Leu Gly Ile Leu Lys Asn Trp Lys Glu Glu Ser


GAC AGA AAA ATA ATG CAG AGC CAA ATT GTC TCC TTT TAC TTC AAA CTT
Asp Arg Lys Ile MET Gln Ser Gln Ile Val Ser Phe Tyr Phe Lys Leu


TTT AAA AAC TTT AAA GAT GAC CAG AGC ATC CAA AAG AGT GTG GAG ACC
Phe Lys Asn Phe Lys Asp Asp Gln Ser Ile Gln Lys Ser Val Glu Thr


ATC AAG GAA GAC ATG AAT GTC AAG TTT TTC AAT AGC AAC AAA AAG AAA
Ile Lys Glu Asp MET Asn Val Lys Phe Phe Asn Ser Asn Lys Lys Lys


CGA GAT GAC TTC GAA AAG CTG ACT AAT TAT TCG GTA ACT GAC TTG AAT
Arg Asp Asp Phe Glu Lys Leu Thr Asn Tyr Ser Val Thr Asp Leu Asn


GTC CAA CGC AAA GCA ATA CAT GAA CTC ATC CAA GTG ATG GCT GAA CTG
Val Gln Arg Lys Ala Ile His Glu Leu Ile Gln Val MET Ala Glu Leu


TCG CCA GCA GCT AAA ACA GGG AAG|CGA AAA AGG AGT CAG ATG
Ser Pro Ala Ala Lys Thr Gly Lys|Arg Lys Arg Ser Gln MET
                                15K


CTG TTT CGA GGT CGA AGA GCA TCC CAG|<u>TAA</u>   X' – ribosome binding
Leu Phe Arg Gly Arg Arg Ala Ser Gln| y'    site and translatio-
                                18k         nal start signal.
                                            y' – translational
                                            stop signal.

# FIG. 3